# EUROPEAN PATENT APPLICATION

(11) **EP 2 110 144 A1**
(43) Date of publication of application: **21.10.2009**
(21) Application number: 08154490.0
(22) Date of filing: 14.04.2008
(51) Int. Cl.: A61L 9/12

(54) **Device for evaporating a fluid that is absorbed by a porous substrate, and method of estimating a level of fluid that is absorbed by a porous substrate**

(71) Applicant: Sara Lee/DE N.V., 3532 AA Utrecht (NL)
(72) Inventor: Atencia, Toni Broncano, 08460 Santa Maria de Palautordera (ES); Bonilla, Francisca-Yolanda Pérez, Castellv'i de Rosanes (ES)
(74) Representative: Hatzmann, Martin

(57) **Abstract**

Method and device for estimating a level of fluid that is absorbed by a porous substrate and a kit, wherein fluid is absorbed by the substrate, wherein a light is arranged near the substrate such that it emits light rays directed to the substrate, wherein the light intensity of the light rays transmitted through the substrate changes under influence of the decrease of the amount of fluid that is absorbed by the substrate.

## Description

The invention relates to a device for a fluid that is absorbed by a porous substrate.

The invention also relates to a method of estimating a level of fluid that is absorbed by a porous substrate.

Known air fresheners oftentimes comprise a bottle holding a porous substrate, such as a wick, for absorbing a volatile air freshening perfume that evaporates into the surroundings. After a certain period of exposing the wick to the environmental air, the amount of perfume that is released into the surroundings by the wick decreases or even ceases to be detectable by a user. To communicate this end of life of the wick to the user, end of life indicators are known that provide for a colour change in the wick when the fluid absorbed by the wick has decreased to a certain level, e.g. has ceased to be detectable. As is described in the international application publication WO2005/118007, a dye that is present in the wick precipitates from the wick when the fluid evaporates, causing a colour change in the wick, and indicating to refill the bottle with perfume.

A disadvantage of such an end of life indicator may be that the wick changes colour unevenly. Also, the addition of the precipitating dye to the components, e.g. the wick or the perfume, may not always be desirable.

It is therefore an object of the invention to provide for an alternative product or manner of indicating a change in an amount of fluid that is absorbed by a porous substrate.

This object and/or other objects may be achieved by a device according to claim 1.

Surprisingly, it has been found that a changing amount of fluid that is absorbed by a porous substrate may change the optical characteristics of the porous substrate. For example, certain porous substrates have different light transmitting properties when being dry, then when a higher amount of fluid is absorbed. In particular, when the porous substrate becomes drier it may become less translucent, while when the porous substrate is wet, it may gain translucency.

To be able to actively indicate a change in the amount of fluid that is absorbed by the porous substrate, a device of the substrate can be provided with a light that shines onto the substrate. The change in intensity of the light that passes through the substrate is an indication of the amount of fluid that is absorbed by the substrate, which change can be detected by a user or a circuit so that a user, or the device itself, can notice that the amount of absorbed fluid has changed and that fluid has to be added to the substrate.

A particular embodiment of the invention uses an active indicator circuit for indicating that the amount of fluid that is absorbed by the porous substrate has changed. For example a light sensor can be provided in the device for detecting an amount of light that is transmitted via the substrate. Additionally, the indicator circuit may signal the change in light intensity that is detected by the sensor to the user.

Abovementioned object and/or other objects may also be achieved by a method according to claim 16.

In clarification of the invention, embodiments thereof will be further elucidated with reference to the drawing. In the drawing:
Figure 1 is a schematic drawing of an air freshener;
Figure 1A a schematical side view of an air freshener;
Figure 2 is a schematic diagram of a freshener.

In this description, identical or corresponding parts have identical or corresponding reference numerals. The exemplary embodiments shown should not be construed to be limitative in any manner and serve merely as illustration.

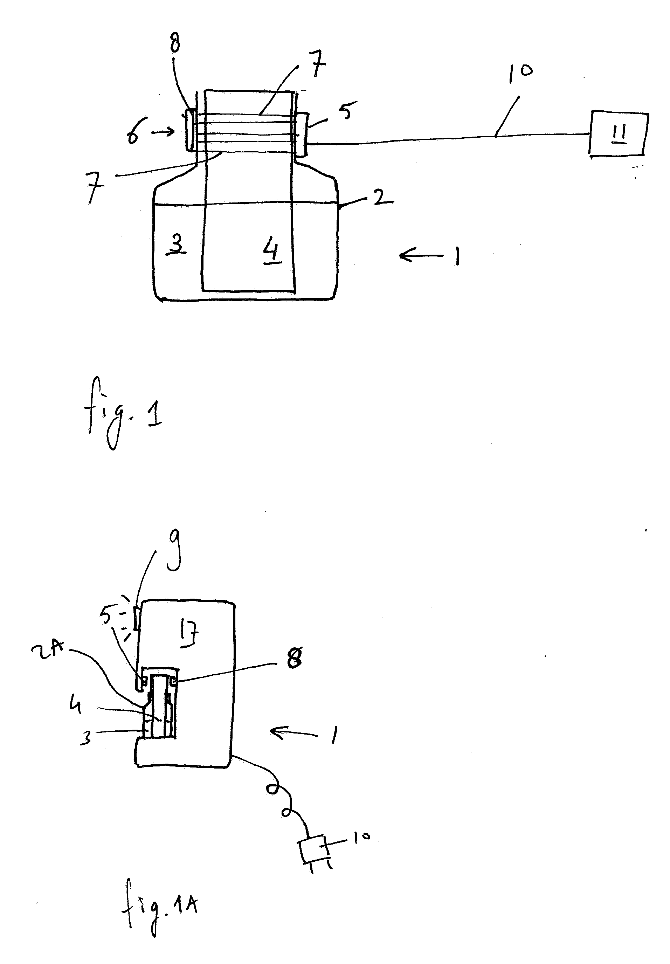

In figure 1 an air freshening device 1, also known as an air freshener or deodoriser, is shown schematically. This device 1 for example comprises a bottle shaped container 2, containing a volatile fluid 3, in particular a perfume, or air freshening or deodorising fluid 3. The container 2 may also hold a porous substrate, in this embodiment a wick 4, which automatically absorbs at least a part of the volatile fluid 3 present in the container 2. The fluid 3 absorbed in the wick 4 evaporates from an exposed end or part of the wick 4 into the environment so that the environment is deodorised, at least as long as fluid 3 is still present in the wick 4. The wick 4 can be of any shape, for example, cylindrical, square shaped, or round shaped.

The device 1 is provided with a lighting circuit 5 that emits light rays 7 onto a wick 4 that is mounted in the device 1, for example placed in the container 2. A connection 10 to a mains power 11 or battery may also be provided to drive the light circuit 5 and also an indicator circuit 6. In use, the light rays 7 emitted by the light circuit 5 pass through the wick 4, at least under certain circumstances as will be explained below, and are received by a light sensor 8 that is provided in an indicator circuit 6.

In an embodiment, the device 1 comprises a housing 17, equipped with said lighting circuit 5, sensor 8, and indicator circuit 6, and arranged for connection with a mains power 11. The container 2, comprising the fluid 3 and the wick 4, can be put in position in the housing 17, while before putting the container 2 in the housing 17, a lid or cap may for example be taken off so that the wick 4 is exposed to air. In another embodiment, when the container 2 is opened by the device 1 itself, during or after being mounted in the housing 17, for example by piercing or otherwise opening the top of the container 2. In again another embodiment, the housing 17 itself may comprise a container 2 that needs to be refilled, while the device 1 automatically keeps the wick 4 wet. If the container 2, or at least the wick 4, is in position, the lighting circuit 5 and the sensor 8 may be arranged near the wick 4, each on one side of the wick 4, when at least the wick 4 is placed in position in the device 1. The housing 17 may be arranged so that it holds the container 2, or at least the wick 4, in position between the sensor 8 and the lighting circuit 5.

In figure 1A an embodiment is shown schematically, comprising a housing 17, wherein refill bottles 2A can be placed for evaporating the fluid 3, for example under influence of electrical heating provided by the housing 17. The lighting circuit 5, e.g. a LED, is arranged at one side of the wick 4, and the light sensor 8 is arranged at the other side of the wick 4, so that light rays emitted by the lighting circuit 5 pass at least partially through the wick 4, at least if the wick 4 is not dry, and light rays are transmitted to the sensor 8. The light rays may be emitted directly onto the wick 4. In an embodiment, the entire bottle 2A, for example including a wick 4, is to be replaced when the fluid 3 has run out, or almost run out. For example, when a bottle 2A is placed in the housing 17, the top of the bottle 2A is opened, for example pierced, so that the fluid 3 therein may evaporate into air. In another embodiment, only the fluid 3 is refilled.

In an embodiment, the indicator circuit 6 is arranged to derive information relating to the amount of fluid 3 in the wick 4, e.g. by comparing the light that is received by the light sensor 8 with certain pre-set values. More specifically, the light sensor 8 converts the received light energy into a signal, which signal is converted by the indicator circuit 6 into a signal relating to the amount of fluid 3 that is present in the wick 4. This information is for example communicated to a user via an indicator 9, which may be a light, as illustrated in fig. 1A.

Preferably, a type of wick 4 is used that has certain optical properties. More preferably, the wick 4 is arranged such that the translucent properties of the wick 4 are influenced by the amount of fluid 3 that is deflected and/or scattered within the wick 4. In particular, the light intensity of the light rays 7 transmitted through such a wick 4 changes under influence of the decrease of the amount of fluid 3 that is absorbed by the wick 4.

For said optical properties, the wick 4 preferably comprises fibres that are transparent or translucent. The refractive index of the material of the wick 4 is preferably relatively close to the refractive index of the fluid 3 that is absorbed. These fibres may be at least partially made of polyolefin. The fibres may for example be partially welded so that a wick 4 is obtained. A fibre of polyolefin may be translucent to light and may have a refractive index of approximately 1,5. Other materials may also be used for the same purpose.

In general, when a number of fibres are welded and/or put together to create a wick 4, the wick 4 may be relatively white and opaque because of the fact that light is largely diffracted by the fibres, at least when the wick 4 is relatively dry. The amount of diffraction may be relatively high when the refractive index of the fluid between the fibres is relatively different from the refractive index of the material of the fibres. For example, when the wick 4 is dried, the fluid between fibres may be air, of which the refractive index is 1, while the refractive index of the fibres may for example be 1,5. This difference is large enough to cause a relatively high amount diffraction. In an embodiment, the refractive index of the material of the wick 4 may be 1,3 or higher, preferably 1,4 or higher, more preferably 1,5 or higher, which is high enough to cause diffraction, at least when the wick 4 is dry.

The diffraction of light in the wick 4 may decrease when the refractive index of the fluid between the fibres is relatively close to the refractive index of the material of the wick 4, or the fibres of the wick. Then more light may be transmitted by the wick 4. For example, when the wick 4 has absorbed a fluid 3, for example a certain perfume, the colour and/or translucency of the wick 4 may change from white and/or opaque, respectively, to translucent and having the characteristics of the colour of the fluid 3, for example the perfume. For example the refractive index of the perfume is around 1,44, which differs substantially from the refractive of air, for example, while it may be relatively close to the refractive index of the material of the wick 4. Preferably, the difference between the refractive index of the fluid 3 and the refractive index of the material of the wick 4 is at least smaller than the difference between the refractive index of the wick 4 and the refractive index of air. For example, the difference between the refractive index of the fluid 3 and the refractive index of the material of the wick 4 is smaller than 0,2, preferably smaller than 0,1.

In an embodiment, the light intensity of the light rays 7 transmitted through the wick 4, received by the light sensor 8, decreases under influence of a decrease in the amount of fluid 3 absorbed by the wick 4. Examples of wicks 4 suitable for such purpose are Filtrona® wicks of the type NWN435179, BNW437108, BNW437604 or BNW435348. Of course, also other wicks 4 can be suitable for such purpose.

In an exemplary embodiment, when there is no wick 4 present in the device 1, the intensity of light received by the sensor 8 may be a relatively large portion of the light emitted by the light source. For example, since there is no element blocking the light rays 7, the light received by the sensor 8 can be between approximately 60% and 100% of the emitted light. However, when a wick 4 is present, the wick 4 will block or at least partly block the light rays 7. For example, if the wick 4 is wet, e.g. soaked with fluid 3, the wick 4 may be relatively translucent. For example, the received light intensity may then be between approximately 5% and approximately 80 %,preferably between 10% and approximately 50% of the intensity of the emitted light. If the wick 4 contains no or little fluid 3, the intensity of the light that is received by the sensor 8 may be significantly lower. It can for example be 0%, or at least relatively low, for example below approximately 20%, or below approximately 10%.

In a practical embodiment, the indicator circuit 6 may be configured to compare the incoming signal from the light sensor 8 with certain pre-set values, for example using a comparator circuit 16, as will be explained later on. For example the indicator circuit 6 will indicate a signal when the signal that is received from the light sensor 8 is around or below a first threshold value. For example, the first threshold is between 1 and approximately 2 Volts, for example 1,5 Volts. When the signal received by the indicator circuit 6 is below 1,5 Volts, the received light intensity may be relatively low because of a relatively dry wick 4, so that the indicator circuit 6 sends a signal that the fluid 3 should be refilled, for example via the indicator 9. For example, a second threshold, which is higher than the first threshold, may have a value of between approximately 3 and approximately 4,5 Volts, preferably around approximately 4 Volts. This may represent a relatively high value relating to an absence of the wick 4, wherein the signal sent by the light sensor relates to a relatively high light intensity. Then, the indicator circuit 6 may send a different signal, indicating that the wick 4 needs to be replaced or a wick has to be placed. Between said first and second thresholds, the wick 4 may contain enough fluid 3 for evaporation, in which case the indicator circuit 6 may or may not send a signal. Of course said thresholds can be of any value, for example a multitude of said values, for example depending on the incoming current and/or the light 12 of the light circuit 5 and/or the indicator circuit 6, etc.

Preferably, the indicator circuit 6 is arranged to compare the light sensor signal with a pre-determined threshold, and drive the indicator 9 when said threshold is reached or crossed. Firstly, the indicator circuit 6 may be arranged to communicate to a user when the wick 4 is dried out, or little fluid 3 is left to be released into the environment. In this way, the user can be actively reminded when no or little fluid is released into the environment. Therefore, in an embodiment the indicator circuit 6 may be arranged to indicate when the light intensity of transmitted light rays 7 received by the light sensor 8 is low, at least below the first threshold for example.

Secondly, the indicator circuit 6 may also be arranged to indicate when the intensity of light that is received by the light sensor 8 is high, or at least above a second threshold that is higher than said first threshold, for example in the case when no wick 4 is present.

Abovementioned thresholds are in fact optional and can be varied, for example depending on the wick 4 and/or fluid 3 that is chosen.

In certain embodiments, the device 1 lasts relatively long, for example for more than 20 days, for example for 30 to 90 days. Sometimes, a user may not be conscious of the fact that the freshener 1 is working, or may not be conscious of the fact that the freshener 1, in particular the wick 4, is out of fluid 3 or almost out of fluid 3. The freshener 1 may for example be inconspicuously placed. Therefore, the indicator 9 may be arranged to indicate to a user a signal relating to an amount of fluid 3 in the wick 4 so that the user may understand that the device 1 can be refilled. For example the indicator 9 provides for a visual signal, for example a light such as a LED, although an audible signal could also be used, when it receives a signal via the indicator circuit 6, e.g. when the freshener does not contain a wick 4 or fluid 3. For example, the user signal of the indicator 9 may be directly related to the received light intensity of the transmitted light. Therefore, in an embodiment, the indicator 9 is a LED or warning light that emits light intermittently when a threshold is reached, and continuously when another threshold is reached. For example, when the wick 4 is not present in the device 1 the indicator 9 emits constantly and the indicator 9 emits intermittently when the amount of fluid is low and the freshener 1 needs a refill. Of course other signals could also be suitable, for example the lighting circuit 5 could change colour, different light symbols could be used, or an LCD screen that presents characters and/or symbols could be used.

In another embodiment, the indicator circuit 6 sends a signal within the device 1 itself. For example, the device 1 may be arranged to switch to a second fluid when a first fluid has run out, the latter being indicated by the indicator circuit 6. For example the device 1 may comprise a housing 17, in use comprising multiple bottles 2A or containers that contain different fluids 3. When one fluid has run out, this is detected by the indicator circuit 6, which sends a signal to a processing circuit that is arranged to activate the device 1 to release the second fluid 3, e.g. by refilling the container that previously contained the first fluid 3, or by opening a second container.

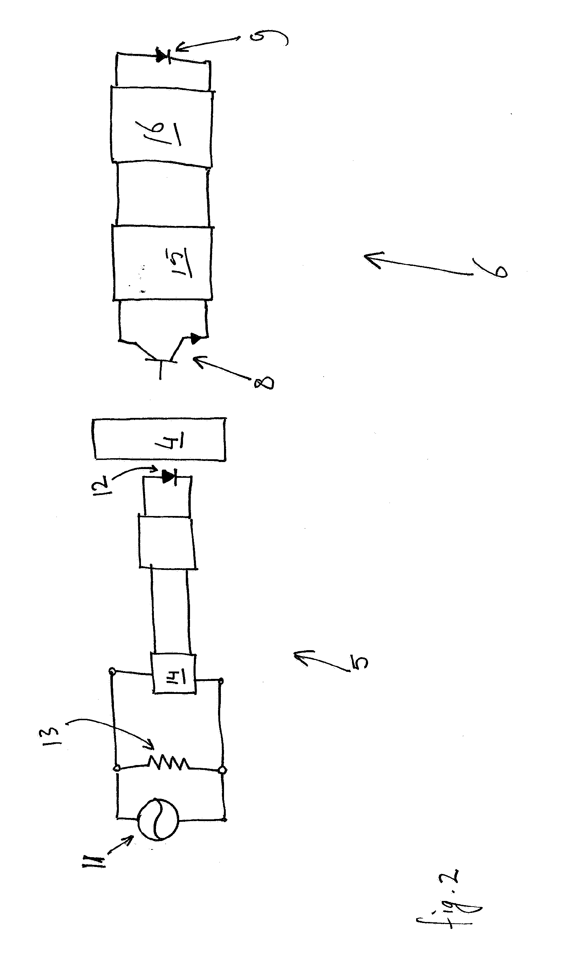

An embodiment of an electrical scheme for a device 1 is shown in figure 2. A lighting circuit 5 is provided at one side of the wick 4 and an indicator circuit 6 is provided at the other side of the wick 4. The lighting circuit 5 is provided with an emitting LED 12 that shines onto the wick 4. The emitting LED 12 is provided with power by a power source, for example a mains power 11 having an oscillating current of approximately 220 to 230 Volts. Optionally, a heating element 13, for example a heating wire, is provided, which heats the wick 4 and/or the fluid 3 therein so that the fluid 3 evaporates more quickly under influence of heat.

In the embodiment of figure 2, an AC/DC converter 14 is provided for outputting a relatively constant signal that is converted from the incoming signal. The AC/DC converter 14 may be switched in parallel with the heating element 13. The converter may provide a 5V constant signal.

A multivibrator, for example an astable 15may be provided that creates holes in the constant signal received from the AC/DC converter 14 so that a time varying signal is achieved. The holes may be created at a certain frequency, for example 10kHz. The emitting LED 12 then emits at the received frequency, for example of 10kHz. This frequency is an example of a frequency that can be distinguished from ambient light, so that noise that comes from ambient light can be filtered, e.g. by the indicator circuit 6, although also other frequencies can be suitable.

In this description, ambient light may be understood as comprising natural sun light, or a reflectance thereof, and the light provided by other light sources in the environment, for example connected to the mains power.

In general, it could be advantageous if the emitted light has certain properties which make it distinguishable from ambient light, since the ambient light may provide for noise that comes into the sensor 8. For example, the light circuit 5 may be arranged to emit at a particular frequency that is lower or higher than 50Hz and/or 60Hz, since a 50Hz/60Hz frequency is a common frequency for mains power lighting systems, although any a frequency different from a standard power source frequency may be suitable to distinguish the light emitted by the lighting circuit 5 from mains power lighting. It could for example also be possible to emit light having a particular colour, e.g. infrared, that can also be distinguished from ambient light.

Turning again to figure 2, the indicator circuit 6 is provided with a light sensor 8 that receives light emitted by the emitting light 12, at least if the wick 4 is at least partly translucent, e.g. is not dry. As already explained above, the wick 4 and light source/sensor arrangement may have three different states. For example, no or little light will be transmitted through the wick 4 if the wick 4 is relatively dry, while a large amount of emitted light will be transmitted to the sensor 8 if no wick 4 is present between the emitting LED 12 and the sensor 8. When the wick 4 is wet, light will at least partially pass through so that the amount of received light exceeds the case of a dry wick 4 and is less than a case wherein no wick 4 is present.

The light sensor 8 receives light and transforms this into an electrical signal. In an embodiment, this signal can be divided in a constant signal, which may be due to ambient light, a time varying signal e.g. at 10kHz, which is due to the transmitted light of the emitting LED 12, and a time varying signal at a different frequency, e.g. 50Hz, 100Hz, 150Hz, etc. which may caused by mains power ambient light. In this description, time varying signals are meant to include oscillating, alternating and pulse signals.

A frequency filter 15 is provided that is arranged to filter out a constant and/or a time varying signal from the incoming signal, in particular the constant signal, and the time varying signal having a frequency different than the frequency of the emitted light, e.g. different from 50Hz. In particular, the filter 15 is arrange to filter out the 50Hz frequency ambient light and the constant ambient light that are received by the sensor 8, such that the 10kHz frequency light of the emitting LED 12 may remain and noise resulting from ambient light is filtered. Hence a signal relating to the wetness of the wick 4 is obtained. The output can for example be a constant signal between 0 and 5 Volts. The output voltage may depend on the amplitude of the time varying voltage that is received at the emitted frequency.

A comparison circuit 16, for example a window comparator circuit, can be switched to the indicator 9. The output of the frequency filter may be received by a comparison circuit 16, which will deliver an output to the indicator 9. For example, if the voltage is less than a first threshold of for example 1,5 Volt, this may mean that the wick is dry or almost dry. If the voltage is more than a second threshold of for example 4 Volts, this may mean that there is no wick 4 in the device 1. If the voltage is between said first and second threshold, for example 1,5 and 4 Volts, respectively, this may imply that there is a wick 4 positioned between the emitting LED 12 and the sensor 8 and it is wet.

Preferably, the comparison circuit 16 will pass a signal to the indicator 9 when there is no wick 4, or when the wick 4 is dry or almost dry, so that the indicator 9 sends a signal indicating that there is no wick 4 in the device 1 and/or the fluid 3 is fully or almost fully evaporated. For example, the indicator 9 comprises a LED or warning light, arranged to shine depending on the output of the comparison circuit 16. For example, the indicator 9 may shine continuously if there is no wick 4, and the indicator 9 may flash if there the freshener 1 has run out of fluid 3.

As explained above, the indicator circuit 6 can be arranged to indicate three different states of the freshener 1 to a user. Likewise the indicator circuit 6 can be arranged to indicate two states, e.g. the wick 4 is wet or the wick 4 is dry, or more than three states, e.g. the wick 4 is wet, the wick 4 is almost dry, the wick 4 is dry, or no wick 4 is present. Also other states relating to the amount of fluid 3 in the wick 4 could also be communicated via the indicator 9.

The volatile fluid 3 can for example be an air freshening or deodorising fluid 3. In other embodiments the fluid 3 is a pesticide composition or a repellent, for example an insect repellent. The fluid 3 may also comprise air moisturiser. The fluid 3 can for example be chosen to cooperate with wick 4 such that the changing light intensity of the wick 4 relative to the amount of absorbed fluid 3 can be readily distinguished.

In an embodiment the wick 4 is enclosed by a container 2, and the fluid 3 will evaporate within the container 2, and the evaporated fluid will spread thereafter via an opening 2 in the container 2.

In an embodiment, the wick 4 itself is used as an indicator 9. For example a lighting circuit 5 emits light onto the wick 4 over a certain period, while within that period the amount of fluid 3 that is absorbed by the wick 4 changes, by evaporation of the fluid 3, so that consequently the translucency of the wick 4 changes. The resulting change of light that is transmitted by the wick 4 can in itself indicate a change in the amount of fluid 3 that is absorbed/evaporated.

It shall be obvious that the invention is not limited in any way to the embodiments that are represented in the description and the drawings. Many variations and combinations are possible within the framework of the invention as outlined by the claims. Combinations of one or more aspects of the embodiments or combinations of different embodiments are possible within the framework of the invention. All comparable variations are understood to fall within the framework of the invention as outlined by the claims.

## Claims

1. Device for evaporating a fluid that is absorbed by a porous substrate, provided with a lighting circuit for emitting light onto said porous substrate when the porous substrate is at least partly mounted in the device.

2. Device according to claim 1, provided with an indicator circuit including:
a light sensor for receiving said light transmitted through said substrate when it is mounted in the device, and converting the received light energy into a signal relating to the received light intensity,
wherein the indicator circuit is arranged to output an indicator signal dependent on the signal from the light sensor relating to the light intensity.

3. Device according to claims 2, wherein the indicator circuit is arranged to indicate said indicator signal when the signal received from the light sensor is around or below a first pre-set threshold value.

4. Device according to claim 3, wherein the indicator circuit is arranged to indicate a signal when the light intensity of light that is received by the light sensor is around or above a certain second threshold that is higher than said first threshold.

5. Device according to claim 3 or 4, wherein the first threshold has a value of between 1 and approximately 2 Volts.

6. Device according to claim 4, wherein the second threshold has a value of between approximately 3 and approximately 4,5 Volts.

7. Device according to any of claims 2 - 6, wherein the indicator circuit comprises a filter that is arranged to filter out a constant and/or a time varying signal.

8. Device according to any of the preceding claims, wherein the lighting circuit is arranged to emit light at a predetermined frequency, which frequency is predetermined so that it can be readily distinguished from the frequencies of ambient light.

9. Device according to claim 8, wherein the frequency of the emitted light is preferably different than 50 or 60 hertz.

10. Device according to any of claims 2 - 9, wherein the indicator circuit comprises a warning light and/or LED for signalling a state of the substrate to a user.

11. Device according to any of the preceding claims, wherein the lighting circuit comprises a LED.

12. Kit of a porous substrate for absorbing fluid and a device according to any of the preceding claims, wherein said substrate is arranged to be mounted between the light sensor and the lighting circuit.

13. Kit according to claim 12, wherein the porous substrate is arranged such that it transmits light when it has absorbed a certain amount of fluid, wherein the light intensity of light transmitted through the substrate is changed when the amount of fluid that is absorbed by the porous substrate is changed.

14. Kit according to claim 12 or 13, wherein the fluid to be evaporated is absorbed by the substrate, wherein the difference between the refractive index of said fluid and the refractive index of the main material of the substrate is smaller than the difference between the refractive index of the main material of the substrate and the refractive index of air

15. Device according to any of claims 2 - 11, or kit according to any of claims 12 - 14, wherein the porous substrate is a wick.

16. Device according to any of claims 2 - 11 or 15, or kit according to any of claims 12 - 15, wherein the porous substrate is a wick for absorbing a perfume or insecticide.

17. Method of estimating a level of fluid that is absorbed by a porous substrate, wherein fluid is absorbed by the substrate, wherein a light is arranged near the substrate such that it emits light rays directed to the substrate, wherein the light intensity of the light rays transmitted through the substrate changes under influence of the decrease of the amount of fluid that is absorbed by the substrate.

18. Method according to claim 17, wherein the transmitted light rays are registered and converted into a signal, which signal is compared to a pre-set threshold, after which, depending on the value of the signal as compared to the threshold, an indicator signal is indicated.

19. Method according to claim 18, wherein the light intensity of the transmitted light rays decreases under influence of the decrease of the amount of fluid that is absorbed by the substrate.
